(19) Europäisches Patentamt
European Patent Office
Office européen des brevets

(11) Veröffentlichungsnummer: **0 452 264 A1**

(12) # EUROPÄISCHE PATENTANMELDUNG

(21) Anmeldenummer: **91810241.9**

(22) Anmeldetag: **03.04.91**

(51) Int. Cl.[5]: **C07C 239/20,** A61K 31/13, C07C 251/58, A61K 31/15, C07D 213/66, A61K 31/44

(30) Priorität: **11.04.90 CH 1248/90**
**02.05.90 CH 1480/90**

(43) Veröffentlichungstag der Anmeldung:
**16.10.91 Patentblatt 91/42**

(84) Benannte Vertragsstaaten:
**AT BE CH DE DK ES FR GB GR IT LI LU NL SE**

(71) Anmelder: **CIBA-GEIGY AG**
**Klybeckstrasse 141**
**CH-4002 Basel (CH)**

(72) Erfinder: **Stanek, Jaroslav, Dr.**
**Hangstrasse 9**
**CH-4144 Arlesheim (CH)**
Erfinder: **Frei, Jörg, Dr.**
**Büchring 36**
**CH-4434 Hölstein (CH)**

(54) **Hydroxylamin-Verbindungen.**

(57) Verbindungen der Formel

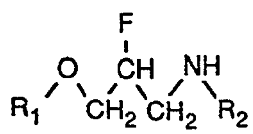

(I),

worin $R_1$ Amino bedeutet oder für einen Rest

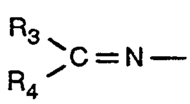

steht, worin $R_3$ Niederalkyl, Hydroxyniederalkyl, Niederalkoxyniederalkyl, Carboxy, Niederalkoxycarbonyl, Phenyl, durch Niederalkyl, Halogenniederalkyl, Hydroxyniederalkyl, Halogen, Hydroxy, Niederalkoxy, Niederalkanoyloxy und/oder Nitro substituiertes Phenyl, Pyridyl, durch Niederalkyl, Hydroxyniederalkyl, Niederalkoxyniederalkyl, Phosphonooxyniederalkyl, Niederalkanoyl, Carboxy, Niederalkoxycarbonyl, Hydroxy, Niederalkoxy, Niederalkanoyloxy, Nitro und/oder Oxido substituiertes Pyridyl oder Chinolyl bedeutet, $R_4$ für Wasserstoff, Niederalkyl, Hydroxyniederalkyl oder Halogenniederalkyl steht oder $R_3$ und $R_4$ zusammen $C_4$-$C_6$-Alkylen oder Benzo-$C_4$-$C_6$-alkylen bedeuten, und $R_2$ geradkettiges $C_1$-$C_4$-Alkyl bedeutet, und Salze davon haben eine starke spezifische Hemmwirkung auf das Enzym Ornithindecarboxylase. Die Verbindungen der Formel I werden nach an sich bekannten Verfahren hergestellt.

EP 0 452 264 A1

Die Erfindung betrifft Verbindungen der Formel

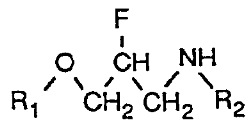

(I),

worin $R_1$ Amino bedeutet oder für einen Rest

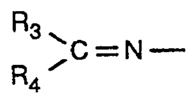

steht, worin $R_3$ Niederalkyl, Hydroxyniederalkyl, Niederalkoxyniederalkyl, Carboxy, Niederalkoxycarbonyl, Phenyl, durch Niederalkyl, Halogenniederalkyl, Hydroxyniederalkyl, Halogen, Hydroxy, Niederalkoxy, Niederalkanoyloxy und/oder Nitro substituiertes Phenyl, Pyridyl, durch Niederalkyl, Hydroxyniederalkyl, Niederalkoxyniederalkyl, Phosphonooxyniederalkyl, Niederalkanoyl, Carboxy, Niederalkoxycarbonyl, Hydroxy, Niederalkoxy, Niederalkanoyloxy, Nitro und/oder Oxido substituiertes Pyridyl oder Chinolyl bedeutet, $R_4$ für Wasserstoff, Niederalkyl, Hydroxyniederalkyl oder Halogenniederalkyl steht oder $R_3$ und $R_4$ zusammen $C_4$-$C_6$-Alkylen oder Benzo-$C_4$-$C_6$-alkylen bedeuten, und $R_2$ geradkettiges $C_1$-$C_4$-Alkyl bedeutet, und ihre Salze, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers und zur Herstellung pharmazeutischer Präparate.

Die vor- und nachstehend verwendeten Allgemeinbegriffe haben im Rahmen der vorliegenden Anmeldung die folgenden Bedeutungen:

Das Präfix "Nieder" bezeichnet einen Rest mit 1 bis 7 und insbesondere mit 1 bis 4 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl, während geradkettiges $C_1$-$C_4$-Alkyl n-Propyl, n-Butyl und insbesondere Ethyl oder Methyl bedeutet.

Hydroxyniederalkyl ist z.B. Hydroxymethyl oder 2-Hydroxyethyl, als Rest $R_3$ ferner auch Polyhydroxyniederalkyl, wie 1,2-Dihydroxyethyl, 1,2,3-Trihydroxypropyl, 1,2,3,4-Tetrahydroxybutyl und insbesondere 1,2,3,4,5-Pentahydroxypentyl. Verbindungen der Formel I, worin $R_1$ für einen Rest $R_3R_4C$=N- steht, $R_3$ Polyhydroxyniederalkyl bedeutet und $R_4$ für Wasserstoff oder Hydroxymethyl steht, leiten sich bevorzugt von Zuckern, insbesondere Aldo- bzw. Keto-(triosen, tetrosen, pentosen oder hexosen) ab. Vor allen Dingen bevorzugt ist dabei der Rest $R_3R_4C$=N-, worin $R_3$1,2,3,4,5-Pentahydroxypentyl (von der D-Glucose abgeleitet) und $R_4$ Wasserstoff bedeutet.

Niederalkoxyniederalkyl ist z.B. Methoxymethyl oder Ethoxymethyl.

Niederalkoxycarbonyl ist z.B. Propoxycarbonyl oder Butoxycarbonyl, vorzugsweise Methoxycarbonyl und Ethoxycarbonyl.

Halogen ist insbesondere Chlor oder Fluor, kann aber auch für Brom stehen.

Halogenniederalkyl ist z.B. Difluormethyl oder Trifluormethyl, ferner 1-Chlorethyl.

Niederalkoxy ist z.B. n-Propoxy, Isopropoxy, n-Butoxy oder tert.-Butoxy, vorzugsweise Ethoxy, und vor allem Methoxy.

Niederalkanoyloxy ist z.B. Formyloxy, Propionyloxy oder Butyryloxy, und vor allem Acetyloxy.

Phosphonoooxyniederalkyl ist z.B. Phosphonooxymethyl [$-CH_2-O-P(=O)(OH)_2$].

Niederalkanoyl ist z.B. Formyl, Propionyl oder Butyryl, und vor allem Acetyl.

Unter einem Rest $R_3R_4C$=N-, worin $R_3$ und $R_4$ zusammen $C_4$-$C_6$-Alkylen bedeuten, ist Cycloalkylidenamino mit 5 bis 7 Ringkohlenstoffatomen, z.B. Cyclopentylidenamino oder Cyclohexylidenamino, zu verstehen.

Unter einem Rest $R_3R_4C$=N-, worin $R_3$ und $R_4$ zusammen Benzo-$C_4$-$C_6$-alkylen bedeuten, ist z.B. Cyclopentylidenamino oder Cyclohexylidenamino zu verstehen, welche jeweils einen annellierten Benzoring tragen.

Substituierte Phenylreste $R_3$ tragen insbesondere einen oder zwei, substituierte Pyridylreste einen bis drei der angegebenen Substituenten. Die angegebenen Substituenten des Pyridinrestes können mit Ausnahme von Nitro, welches nur an einem Ringkohlenstoffatom gebunden sein kann, und Oxido, welches nur an einem Ringstickstoffatom gebunden sein kann, sowohl an Ringkohlenstoffatomen als auch an Ringstickstoffatomen gebunden sein. Pyridyl steht für z.B. 2-, 3- oder 4-Pyridyl, Chinolinyl z.B. für 2- oder 4-Chinolyl.

Salze von erfindungsgemässen Verbindungen sind in erster Linie pharmazeutisch annehmbare, nicht-toxische Salze. Beispielsweise können Verbindungen der Formel I mit basischen Gruppen Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure, Oxalsäure oder Methansulfonsäure, oder z.B. mit Aminosäuren, wie Glutaminsäure, bilden. Bei Anwesenheit von mehreren basischen Gruppen können Mono- oder Polysalze gebildet werden. Verbindungen der Formel I mit einer sauren Gruppe, z.B. Carboxy, und einer basischen Gruppe, z.B. Amino, können z.B. in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch annehmbaren, nicht-toxischen Salze, die deshalb bevorzugt sind.

Die Verbindungen der vorliegenden Erfindung können in Form von Isomerengemischen oder von reinen Isomeren, ferner als Racemate oder als optisch aktive Verbindungen, vorliegen.

Die erfindungsgemässen Verbindungen weisen wertvolle, insbesondere pharmakologisch verwendbare, Eigenschaften auf. Insbesondere haben sie eine starke, spezifische Hemmwirkung auf das Enzym Ornithindecarboxylase (ODC). ODC spielt als ein Schlüsselenzym eine wichtige Rolle bei der Polyaminbiosynthese, die in praktisch allen Zellen von Säugetieren, einschliesslich Menschen, abläuft. Durch ODC wird die Polyaminkonzentration in der Zelle reguliert. Eine Hemmung des Enzyms ODC hat eine Verringerung der Polyaminkonzentration zur Folge. Da eine Verringerung der Polyaminkonzentration eine Hemmung des Zellwachstums bewirkt, ist es möglich, durch Verabreichung von ODC-hemmenden Substanzen das Wachstum sowohl von eukaryotischen als auch prokaryotischen, inbesondere von rasch oder unkontrollierbar wachsenden, Zellen zu hemmen und sogar Zellen abzutöten oder das Einsetzen der Zelldifferenzierung zu hemmen.

Die Hemmung des Enzyms ODC kann z.B. mit der Methode von J.E. Seely und A.E. Pegg, Ornithin decarboxylase (mouse kidney), Seiten 158-161, in H. Tabor und C. White Tabor (Hrsg.): Methods in Enzymology, Vol. 94: Polyamines, Academic Press, New York 1983, nachgewiesen werden.

Die Verbindungen der Formel I besitzen antiproliferative Eigenschaften, die sich z.B. in dem folgenden Versuch direkt demonstrieren lassen: Dabei wird die Hemmwirkung der Verbindungen der Formel I auf das Wachstum von menschlichen T24 Blasenkarzinomzellen bestimmt. Diese Zellen werden in "Eagle's minimal essential medium", dem 5 % (V/V) fötales Kälberserum zugesetzt sind, in einem befeuchteten Inkubator bei 37°C und 5 Volumenprozent $CO_2$ in der Luft inkubiert. Die Karzinomzellen ( 1000-1500) werden in 96-Loch-Mikrotiterplatten überimpft und über Nacht unter den obengenannten Bedingungen inkubiert. Die Testsubstanz wird in seriellen Verdünnungen am Tag 1 hinzugefügt. Die Platten werden unter den obengenannten Bedingungen 5 Tage lang inkubiert. Während dieser Zeitspanne durchlaufen die Kontrollkulturen mindestens 4 Zellteilungen. Nach der Inkubation werden die Zellen mit 3,3%iger (Gewicht/Volumen) wässriger Glutaraldehydlösung fixiert, mit Wasser gewaschen und mit 0,05%iger (G/V) wässriger Methylenblaulösung gefärbt. Nach dem Waschen wird der Farbstoff mit 3%iger (G/V) wässriger Salzsäure eluiert. Danach wird die optische Dichte (OD) pro Loch, welche der Zellanzahl direkt proportinal ist, mit einem Photometer (Titertek multiskan) bei 665 nm gemessen. Die $IC_{50}$-Werte werden mit einem Computersystem unter Verwendung der Formel

$$\frac{OD_{665}\,(\text{Test}) - OD_{665}\,(\text{Anfang})}{OD_{665}\,(\text{Kontrolle}) - OD_{665}\,(\text{Anfang})} \times 100$$

errechnet. Die $IC_{50}$-Werte sind als diejenige Wirkstoffkonzentration definiert, bei der die Anzahl der Zellen pro Loch am Ende der Inkubationszeit nur 50 % der Zellanzahl in den Kontrollkulturen beträgt.

Die Verbindungen der Formel I sind daher z.B. geeignet zur Behandlung benigner und maligner Tumoren. Sie können Tumorregressionen bewirken und ferner die Verbreitung von Tumorzellen sowie das Wachstum von Mikrometastasen Verhindern. Des weiteren können sie z.B. zur Behandlung von Protozoainfektionen, wie etwa Trypanosomiasis, Malaria oder durch Pneumocystis carinii verursachte Lungenentzündung, dienen.

Insbesondere betrifft die Erfindung Verbindungen der Formel I, worin $R_1$ Amino bedeutet oder für einen Rest

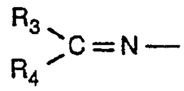

steht, worin $R_3$ Niederalkyl, Hydroxyniederalkyl, Niederalkoxyniederalkyl, Phenyl, durch Hydroxy substituiertes Phenyl, Pyridyl, durch Hydroxy, Niederalkyl, Hydroxyniederalkyl und/oder Phosphonooxyniederalkyl substituiertes Pyridyl, oder Chinolyl bedeutet, $R_4$ Wasserstoff, Niederalkyl oder Halogenniederalklyl bedeutet, oder $R_3$ und $R_4$ zusammen für $C_4$-$C_6$-Alkylen oder Benzo-$C_4$-$C_6$-alkylen stehen, und $R_2$ geradkettiges $C_1$-$C_4$-Alkyl bedeutet, und Salze davon.

In erster Linie betrifft die Erfindung Verbindungen der Formel I, worin $R_1$ Amino bedeutet oder für einen Rest

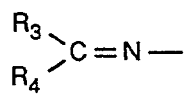

steht, worin $R_3$ Niederalkyl, durch Hydroxy substituiertes Phenyl oder durch Hydroxy, Niederalkyl, Hydroxyniederalkyl und/oder Phosphonooxyniederalkyl substituiertes Pyridyl bedeutet, $R_4$ Wasserstoff oder Niederalkyl bedeutet, und $R_2$ geradkettiges $C_1$-$C_4$-Alkyl bedeutet, und Salze davon.

Bevorzugt betrifft die Erfindung Verbindungen der Formel I, worin $R_1$ Amino bedeutet und $R_2$ geradkettiges $C_1$-$C_4$-Alkyl bedeutet, und Salze davon.

Die Erfindung betrifft vor allem die in den Beispielen beschriebenen spezifischen Verbindungen und pharmazeutisch annehmbare Salze davon.

Die neuen Verbindungen der Formel I können in an sich bekannter Weise hergestellt werden, indem man z.B.

(a) eine Verbindung der Formel II

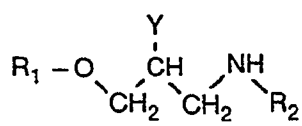

(II)

worin $R_1$ und $R_2$ die unter Formel I angegebene Bedeutung haben und Y eine in Fluor überführbare oder durch Fluor austauschbare Gruppe ist, mit einem Fluorierungsmittel umsetzt, oder

(b) zur Herstellung von Verbindungen der Formel I, worin $R_1$ Amino bedeutet, in einer Verbindung der Formel

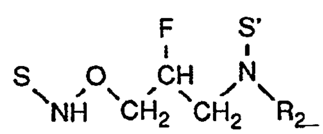

(III)

worin $R_2$ die unter Formel I angegebene Bedeutung hat und S und S' unabhängig voneinander für eine Aminoschutzgruppe oder Wasserstoff stehen, wobei mindestens eine der Gruppen S und S'eine Aminoschutzgruppe bedeutet, die Aminoschutzgruppe(n) abspaltet, oder

(c) zur Herstellung von Verbindungen der Formel I, worin $R_1$ Amino bedeutet, eine Verbindung der Formel

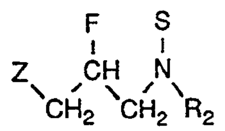

(IV)

worin $R_2$ die unter Formel I angegebene Bedeutung hat, Z Hydroxy oder eine nucleofuge Abgangsgruppe bedeutet und S eine Aminoschutzgruppe oder Wasserstoff bedeutet, mit gegebenenfalls N-geschütztem Hydroxylamin umsetzt und gegebenenfalls die Aminoschutzgruppe abspaltet, oder
(d) eine Verbindung der Formel

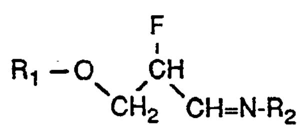

(V)

worin $R_1$ und $R_2$ die unter Formel I angegebene Bedeutung haben, reduziert; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung in ein Salz umwandelt, und/oder ein erhaltenes Gemisch von isomeren Verbindungen der Formel I in die einzelnen Isomeren auftrennt.

In der folgenden näheren Beschreibung der Verfahren (a)-(d) haben die Symbole $R_1$ und $R_2$ jeweils die unter Formel I angegebene Bedeutung, sofern nichts anderes angegeben ist.

Verfahren (a): Eine in Fluor überführbare oder durch Fluor austauschbare Gruppe Y ist z.B. Hydroxy, Halogen (Chlor, Brom, Jod) oder aliphatisch- oder aromatisch-substituiertes Sulfonyloxy, z.B. Methylsulfonyloxy oder 4-Methylphenylsulfonyloxy (Tosyloxy). Vorzugsweise bedeutet Y Hydroxy.

Fluorierungmittel, die geeignet sind, Hydroxy in Fluor zu überführen, sind z.B. Fluorwasserstoff, Schwefeltetrafluorid, insbesondere ein Gemisch aus Fluorwasserstoff und Schwefeltetrafluorid und ferner auch z.B. substituierte Aminoschwefeltrifluoride, wie Diethylamino-schwefeltrifluorid (DAST) oder Piperidino-schwefeltrifluorid.

Verbindungen der Formel II, worin Y Arylsulfonyloxy, z.B. Tosyloxy, oder Halogen, wie Chlor, Brom oder Jod, bedeutet, können ferner z.B. durch Umsetzung mit $KHF_2$, etwa in 1,2-Dihydroxyethan, in die Verbindungen der Formel I umgewandelt werden.

Insbesondere bei der zuletzt genannten Umsetzung kann es erforderlich sein, die Aminogruppen in den Verbindungen der Formel II vor der Durchführung der Reaktion durch Aminoschutzgruppen, z.B. die unten genannten, zu schützen. Nach der Reaktion werden die Aminoschutzgruppen dann in an sich bekannter Weise wieder abgespalten.

Die Ausgangsverbindungen der Formel II, worin Y für Hydroxy steht, werden z.B hergestellt, indem man eine Verbindung der Formel

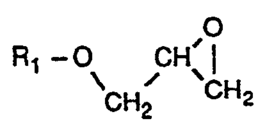

(VI)

worin $R_1$ die unter Formel I angegebene Bedeutung hat, mit einem Amin der Formel $R_2NH_2$, worin $R_2$ die unter Formel I angegebene Bedeutung hat, umsetzt. Die Ausgangsverbindungen der Formel II, worin Y aliphatisch- oder aromatisch-substituiertes Sulfonyloxy bedeuten, werden bevorzugt in an sich bekannter Weise aus Verbindungen der Formel II, worin Y für Hydroxy steht, hergestellt, z.B. durch Reaktion mit Niederalkylsulfonyl- oder Arylsulfonylchloriden.

Die Umsetzung des Epoxids der Formel VI mit dem Amin der Formel $NH_2R_2$ wird ohne Lösungsmittel oder in Gegenwart eines Lösungsmittels, z.B. einem Niederalkanol oder Ether, wie Isopropanol oder Tetrahydrofuran, und gegebenenfalls unter erhöhtem Druck durchgeführt und erfolgt selektiv am terminalen C-Atom des Oxiranylrestes.

Die Ausgangsverbindungen der Formel VI werden z.B. dadurch erhalten, dass man ein Hydroxylamin bzw.

Oxim der Formel $R_1$-OH mit z.B. Epichlorhydrin, Epibromhydrin oder 3-Tosyloxy-1,2-epoxypropan umsetzt. Diese Reaktion wird bevorzugt in Gegenwart einer Base, z.B. Natriumhydroxid, und ohne Lösungsmittel oder in Gegenwart eines Lösungsmittels, z.B. Aceton oder Acetonitril, durchgeführt.

Setzt man bei der Herstellung von Verbindungen der Formel VI optisch aktives Epichlorhydrin, Epibromhydrin oder insbesondere 3-Tosyloxy-1,2-epoxypropan ein, so erhält man stereoselektiv die entsprechenden optisch aktiven Verbindungen der Formel VI. Werden letztere eingesetzt, so gelangt man zu optisch aktiven Verbindungen der Formel II.

Verfahren (b): Bevorzugte monovalente Aminoschutzgruppen S und S' sind Estergruppen, z.B. Niederalkylester und insbesondere tert.-Butoxycarbonyl (BOC), Acylreste, z.B. Niederalkanoyl oder Halogenniederalkanoyl, wie insbesondere Acetyl, Chloracetyl oder Trifluoracetyl oder eine aliphatisch oder aromatisch substituierte Sulfonylgruppe, z.B. Methansulfonyl oder Toluolsulfonyl (Tosyl).

In Verbindungen der Formel III kann S auch eine bivalente Aminoschutzgruppe darstellen. Bevorzugte bivalente Aminoschutzgruppen S sind mono- oder disubstituierte Methylidengruppen, wie $=C(CH_3)_2$ oder =CH-Phenyl, ferner 1-Niederalkoxy (beispielsweise Methoxy oder Ethoxy)-niederalkyliden (beispielsweise Ethyliden oder 1-n-Butyliden), z.B. $=C(CH_3)(OC_2H_5)$ oder Bisacylreste, z.B. der Phtalylrest, welcher zusammen mit dem zu schützenden Stickstoffatom ein 1H-Isoindol-1,3(2H)-dion (Phtalimidogruppe) bildet.

Die Wirkungsweise von Schutzgruppen, z.B. Aminoschutzgruppen, ihre Einführung und Abspaltung sind an sich bekannt und z.B. in J.F.W. McOmie, "Protective Groups in Organic Chemistry", Plenum Press, London and New York 1973, und T.W. Greene, "Protective Groups in Organic Synthesis", Wiley, New York 1984 beschrieben.

Die Abspaltung der Aminoschutzgruppen kann z.B. hydrolytisch, insbesondere im sauren Medium, z.B. mit Chlorwasserstoff, verdünnter Schwefelsäure, Oxalsäure, organischen Sulfonsäuren, z.B. Toluol-4-sulfonsäure, oder Trifluoressigsäure, geschehen.

Verbindungen der Formel III sind bekannt oder werden nach an sich bekannten Verfahren hergestellt.

Verbindungen der Formel III werden z.B. dadurch hergestellt, dass man z.B. eines der Verfahren (a), (c) oder (d) mit geschützter (geschützten) Aminogruppe(n) durchführt. Ferner können Verbindungen der Formel III auch aus Verbindungen der Formel I - etwa zu Reinigungszwecken - hergestellt werden.

Verfahren (c): In den Verbindungen der Formel IV ist die nucleofuge Abgangsgruppe Z z.B. Halogen, z.B. Chlor, Brom oder Iod, ferner aber auch z.B. aliphatisch- oder aromatisch-substituiertes Sulfonyloxy, z.B. Methylsulfonyloxy oder 4-Methylphenylsulfonyloxy. Die Aminoschutzgruppen S in einer Verbindung der Formel IV entsprechen den in Verbindungen der Formel III unter Verfahren (b) definierten Aminoschutzgruppen S bzw. S'.

Bedeutet in einer Verbindung der Formel IV Z Hydroxy, so kann man z.B. eine intermolekulare Dehydratisierungsreaktion durchführen. Insbesondere kommt dafür eine Variante der Mitsunobu-Reaktion [Synthesis (1976), 682] in Frage, bei der die Verbindung der Formel IV mit einem N-geschützten Hydroxylamin-Derivat, wie z.B. N-Hydroxyphthalimid, N-Hydroxy-5-norbornen-2,3-dicarbonsäureimid oder Acethydroxamsäureethylester, und z.B. Triphenylphosphin und N,N'-Azodicarbonsäurediethylester umgesetzt wird.

Bedeutet in einer Verbindung der Formel IV Z eine nucleofuge Abgangsgruppe, so entspricht das Verfaren (c) einer einfachen nucleophilen Substitution (O-Alkylierung).

Verfahren (d): Die Reduktion kann z.B. mit komplexen Metallhydriden, z.B. $LiBH_4$, $NaCNBH_3$, oder mit Wasserstoff in Gegenwart eines Katalysators, z.B. Palladium auf Kohle, durchgeführt werden.

Die Ausgangsverbindungen der Formel V lassen sich z.B. aus den analogen Aldehyden (=O statt $=NR_2$ in Formel V) durch Umsetzung mit Niederalkylaminen herstellen.

Die erwähnten Aldehyde analog zur Formel V lassen sich z.B. durch Oxidation, z.B. mit Pyridiniumchlorchromat [vgl. J. Org. Chem. 46, 4797 (1981)], aus entsprechenden Hydroxymethylverbindungen herstellen. Weiterhin sind sie auch durch Reduktion von entsprechenden Niederalkylestern, z.B. mit Diisobutylaluminiumhydrid [vgl. Chem. Pharm. Bull. 23, 3081 (1975)], oder durch Reduktion von entsprechenden Säurechloriden, z.B. mit Tri-n-butylzinnhydrid [vgl. J. Org. Chem. 25, 284 (1961) oder J. Amer. Chem. Soc. 88, 571 (1966)] erhältlich.

Die erwähnten Aldehyde analog zur Formel V können insbesondere auch über die folgende Reaktionssequenz erhalten werden:

(1) Umsetzung von 3,4-0,0-Isopropyliden-3,4-dihydroxy-1,2-epoxybutan [s. J. Org. Chem. 52, 2841 (1987) oder DE-A-3,150,917] mit Acethydroxamsäureethylester zum 3,4-0,0-Isopropyliden-2,3,4-trihydroxy-1-(1-ethoxyethylidenaminoxy)-butan. In letzterem kann die 2-Hydroxygruppe in an sich bekannter Weise in Fluor umgewandelt werden.

(2) Abspaltung der Isopropylidengruppe z.B. durch Behandlung mit verdünnter Säure.

(3) Glykolspaltung der endständigen $\alpha,\beta$-Dihydroxyethylgruppe zu Formyl durch Umsetzung mit $NaIO_4$ oder $Pb(OCOCH_3)_4$.

Verbindungen der Formel I können in andere Verbindungen der Formel I umgewandelt werden.

Verbindungen der Formel I, worin $R_1$ Amino bedeutet, lassen sich durch Umsetzung mit einer Carbonylverbindung $R_3R_4C{=}O$, worin die Carbonylgruppe auch maskiert vorliegen kann - etwa als Acetal oder Ketal -, in andere Verbindungen der Formel I überführen, worin $R_1$ für einen Rest

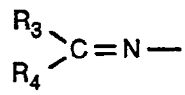

steht.

Umgekehrt können auch Verbindungen der Formel I, worin $R_1$ für einen Rest

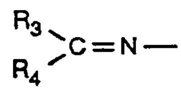

steht, z.B. durch saure Hydrolyse in Verbindungen der Formel I umgewandelt werden, worin $R_1$ Amino bedeutet.

Verfahrensgemäss erhältliche freie Verbindungen der Formel I mit salzbildenden Eigenschaften können in an sich bekannter Weise in ihre Salze übergeführt werden, Verbindungen mit basischen Eigenschaften durch Behandeln mit Säuren oder geeigneten Derivaten davon.

Die Verbindungen, einschliesslich ihrer Salze, können auch in Form ihrer Hydrate erhalten werden, oder ihre Kristalle können z.B. das zur Kristallisation verwendete Lösungsmittel einschliessen.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Tempe- ratur, z.B. im Temperaturbereich von etwa -80°C bis etwa 190°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

In die Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und

das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragées-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,3 g bis etwa 15 g, vorzugsweise von etwa 0,5 g bis etwa 5 g einer Verbindung der vorliegenden Erfindung verabreicht.

Die nachfolgenden Beispiele illustrieren die vorliegende Erfindung; Temperaturen werden in Grad Celsius angegeben. Folgende Abkürzugen werden verwendet: Essigester ≙ Essigsäureethylester, BOC ≙ tert.-Butyloxycarbonyl; Hexan ≙ n-Hexan; Ether ≙ Diethylether, THF ≙ Tetrahydrofuran.

Beispiel 1: N-(3-Aminoxy-2-fluorpropyl)-methylamin-dihydrochlorid

3,3 g N,N'-Di-BOC-N-(3-Aminoxy-2-fluorpropyl)-methylamin werden in 5 ml Ethanol gelöst, mit 35 ml 2N alkoholischer Salzsäure versetzt und 16 h bei Raumtemperatur stehen gelassen. Anschliessend werden 100 ml trockener Ether zugegeben. Die ausgeschiedene Titelverbindung wird abgesaugt, mit Ether gewaschen und getrocknet, Smp. 160° (mit Zers.).

$^1$H-NMR ($D_2O$): δ 5,3 und 5,15 (2m, 1H); 4,38 (m, 2H); 3,45 (m, 2H); 2,8 (s, 3H).

Die Ausgangsverbindung wird wie folgt hergestellt:

Eine Lösung von 8,0 g (50 mMol) O-(2,3-Epoxypropyl)-acethydroxamsäureethylester und 50 ml einer 33 %igen ethanolischen Methylaminlösung in 100 ml Isopropanol werden 4 Stunden bei 85° gerührt und zur Trockene eingedampft. Der erhaltene Rückstand wird über 250 g Kieselgel mit Essigester chromatographiert. Die Fraktionen mit dem Rf-Wert 0,21 (Kieselgel/Methylenchlorid:Methanol:konz. Ammoniak 150:50:1) werden vereinigt und eingedampft. Das zurückgebliebene gelbe Oel stellt den O-(3-Methylamino-2-hydroxypropyl)-acethydroxamsäure-ethylester dar.

Ein Gemisch von 6,5 g (34 mMol) O-(3-Methylamino-2-hydroxypropyl)-acethydroxamsäure-ethylester in 150 ml 2N Salzsäure wird 4 Stunden am Rückfluss gekocht und anschliessend zur Trockene eingedampft. Der wachsartige Rückstand von N-(3-Aminoxy-2-hydroxypropyl)-methylamin-dihydrochlorid wird in wenig Wasser aufgenommen, durch Filtration über 150 ml Dowex 1x4 Ionenaustauscherharz (in basischer Form) in die freie Base umgewandelt und als Oxalatsalz kristallisert, Smp. 130-133° (aus Methanol), Rf-Wert 0,43 (Kieselgel/Methylenchlorid:Methanol:konz. Ammoniak 40: 10:1).

4,8 g (25 mMol) N-(3-Aminoxy-2-hydroxypropyl)-methylamin-dihydrochlorid werden bei -78° in einem Teflonautoklav in 80 g flüssigen Fluorwasserstoff gelöst. Man leitet 5,6 g Schwefeltetrafluorid ein und lässt das Gefäss 24 h verschlossen bei Raumtemperatur stehen. Nach dem Entgasen wird der Rückstand in 2N Salzsäure gelöst. Diese Lösung wird filtriert, das Filtrat portionsweise mit festem Natriumbicarbonat neutralisiert, mit einer Lösung von 13 g Di-tert.-butyldicarbonat in 50 ml Tetrahydrofuran versetzt und 1 h stehengelassen. Das Reaktionsgemisch wird mit 200 ml Ether verdünnt und die organische Phase abgetrennt, mit Wasser gewaschen, über Magnesiumsulfat getrocknet, filtriert und eingedampft. Das zurückgebliebene Oel wird mit einem Gemisch von Hexan:Essigester 3:1 über 500 g Kieselgel chromatographiert und liefert die Ausgangsverbindung als gelbes Oel, Rf-Wert 0,18.

Beispiel 2 : N-[3-(2-Hydroxy-benzylidenaminoxy)-2-fluorpropyl]-methylaminhydrochlorid

Eine Lösung von 488 mg (2,5 mMol) N-(3-Aminoxy-2-fluorpropyl)-methylamin-dihydrochlorid in 10 ml Ethanol wird mit 2,5 ml 1N Natronlauge und 305 mg (2,5 mMol) Salicylaldehyd versetzt und 24 h bei Raumtemperatur gerührt. Danach wird das Relationsgemisch zur Trockne eingedampft und der Rückstand aus Ethanol/Ether kristallisiert.

$^1$H-NMR ($D_2O$): δ 8,45 (s, 1H); 7,4 (m, 2H); 7,0 (m, 2H); 5,28 und 5,07 (2m, 1H); 4,46 (m, 2H); 3,41 (m, 2H); 2,82 (s, 3H).

Beispiel 3:
N-[3-(3-Hydroxy-5-hydroxymethyl-2-methyl-4-pyridylmethylenaminoxy)-2-fluorpropyl]-methylamin-hydrochlorid

Eine Lösung von 390 mg (2,0 mMol) N-(3-Aminoxy-2-fluorpropyl)-methylamin-dihydrochlorid in 10 ml Ethanol wird mit 4,0 ml 1 N Natronlauge und 408 mg (2 mMol) Pyridoxal-hydrochlorid versetzt und 16 h bei Raumtemperatur gerührt. Danach wird das Relationsgemisch zur Trockne eingedampft und die Titelverbindung aus Essigester kristallisiert.

$^1$H-NMR ($D_2O$): δ 8,8 (s, 1H); 8,2 (s, 1H); 5,33 und 5,12 (2m, 1H); 4,86 (s, 2H); 4,65 (m, 2H); 3,42 (m, 2H); 2,78 (s, 3H); 2,66 (s, 3H).

Beispiel 4: N-(3-Isopropylidenaminoxy-2-fluorpropyl)-methylamin-hydrochlorid

Eine Lösung von 488 mg (2,5 mMol) N-(3-Aminoxy-2-fluorpropyl)-methylamin-dihydrochlorid in 10 ml Ethanol wird mit 2,5 ml 1N Natronlauge und 0,36 ml (5 mMol) Aceton versetzt und 24 h bei Raumtemperatur gerührt. Danach wird das Reaktionsgemisch zur Trockne eingedampft. Der Rückstand wird in Ethanol aufgenommen, filtriert und durch Zusatz von Ether kristallisiert.

$^1$H-NMR ($D_2O$): 5,3 und 5,11 (2m, 1H); 4,44 (m, 2H); 3,39 (m, 2H); 2,01 (s, 6H).

Beispiel 5: N-(3-Aminoxy-2-fluorpropyl)-methylamin-dihydrochlorid

1,45 g (3,8 mMol) N-(3-BOC-Aminoxy-2-fluorpropyl)-N-methyl-4-toluolsulfonamid werden in 10 ml Wasser und 20 ml konz. Salzsäure suspendiert und 6 h am Rückfluss erhitzt. Nach dem Abkühlen wird das Reaktionsgemisch filtriert und mit Essigester extrahiert. Die wässrige Phase wird dann zur Trockne eingedampft und die Titelverbindung aus Ethanol/Ether kristallisiert, Smp. 160°C (mit Zers.).

$^1$H-NMR ($D_2O$): δ 5,3 und 5,15 (2m, 1H); 4,38 (m, 2H); 3,45 (m, 2H); 2,8 (s, 3H).

Die Ausgangsverbindungen werden wie folgt hergestellt:

(a) 3-Aminoxy-2-fluorpropylamin-dihydrochlorid

In einem 200 ml Teflon-Reaktor werden 2,5 g 3-Aminoxy-2-hydroxypropylamin (s. DE 2 651 083) bei -78° in 40 g flüssigem Fluorwasserstoff (HF) gelöst. Anschliessend werden noch 5,6 g Schwefeltetrafluorid eingeleitet. Das Gemisch im Reaktor wird verschlossen 3 h bei -78° mit einem Magnetstab gerührt, auf 0° erwärmt und nach weiteren 24 h entgast. Man erhält so das rohe 3-Aminoxy-2-fluorpropylamin als Hydrofluorid, das wie folgt in das entsprechende reine Dihydrochlorid überführt wird:

Der Rückstand wird in 50 ml 2N HCl aufgenommen, filtriert und auf eine 35 x 270 mm Säule mit schwach basischem Ionenaustauscher MWA-1 (Dow Chemicals) aufgetragen. Die Säule wird mit Wasser gewaschen. Die Ninhydrin-positiven Fraktionen werden vereinigt, mit 1N HCl neutralisiert und eingedampft. Der Rückstand wird aus Essigester kristallisiert, und man erhält 3-Aminoxy-2-fluorpropylamin-dihydrochlorid vom Smp. 204-207°.

(b) 3-BOC-Aminoxy-2-fluorpropylamin

Eine Lösung von 362 mg (2 mMol) 3-Aminoxy-2-fluorpropylamin-dihydrochlorid in 25 ml THF, 5 ml Wasser und 2 ml 1N NaOH wird mit einer Lösung von 480 mg (2,2 mMol) Di-tert.-butyldicarbonat in 10 ml THF versetzt und 16 h bei Raumtemperatur kräftig gerührt. Das Reaktionsgemisch wird mit 50 ml Ether und weiteren 2 ml 1N NaOH verdünnt. Die organische Phase wird abgetrennt, mit Wasser gewaschen, über Natriumsulfat getrocknet und eingedampft. Man erhält so die Ausgangsverbindung a als gelbes Oel.

(c) N-(3-BOC-Aminoxy-2-fluorpropyl)-4-toluolsulfonamid

Eine Lösung von 0,52 g (2,5 mMol) 3-BOC-Aminoxy-2-fluorpropylamin und 0,42 ml (3 mMol) Triethylamin in 10 ml Methylenchlorid wird unter Rühren und Eiskühlung tropfenweise mit einer Lösung von 0,48 g (2,5 mMol) Toluol-4-sulfochlorid versetzt und 16 h bei Raumtemperatur gerührt. Das Reaktionsgemisch wird danach mit 25 ml 0,2N Salzsäure und mit Wasser gewaschen, über Magnesiumsulfat getrocknet, filtriert und eingedampft. Der Rückstand stellt die Ausgangsverbindung b dar.

(d) N-(3-BOC-Aminoxy-2-fluorpropyl)-N-methyl-4-toluolsulfonamid

0,54 g (1,5 mMol) N-(3-BOC-Aminoxy-2-fluorpropyl)-4-toluolsulfonamid werden in 10 ml Ethanol und 1,5 ml 1N Natronlauge gelöst und nach der Zugabe von 0,21 g (1,5 mMol) Methyljodid 10 h im Bombenrohr bei 70° gehalten. Nach dem Abkühlen wird das Reaktionsgemisch eingedampft und der Rückstand in Essigester aufgenommen. Man wäscht diese Lösung mit Wasser, trocknet über Magnesiumsulfat und erhält die Ausgangsverbindung c nach Abdampfen des Lösungsmittels.

Beispiel 6: N-(3-Aminoxy-2-fluorpropyl)-ethylamin-dihydrochlorid

Analog Beispiel 5 wird ausgehend von N-(3-BOC-Aminoxy-2-fluorpropyl)-4-toluolsulfonamid und Ethylbromid die Titelverbindung hergestellt.
$^1$H-NMR ($D_2O$): δ 5,31 und 5,15 (2m, 1H); 4,39 (m, 2H); 3,48 (m, 2H); 3,30 (m, 2H); 1,25 (t, 3H).

Beispiel 7: N-(3-Aminoxy-2-fluorpropyl)-propylamin-dihydrochlorid

Analog Beispiel 5 wird ausgehend von N-(3-BOC-Aminoxy-2-fluorpropyl)-4-toluolsulfonamid und n-Propyljodid die Titelverbindung hergestellt.
$^1$H-NMR ($D_2O$): δ 5,29 und 5,06 (2m, 1H); 4,43 (m, 2H); 3,21-3,5 (m, 6H); 1,21 (t, 3H).

Beispiel 8: N-(3-Aminoxy-2-fluorpropyl)-n-butylamin-dihydrochlorid

Analog Beispiel 5 wird ausgehend von N-(3-BOC-Aminoxy-2-fluorpropyl)-4-toluolsulfonamid und n-Butyljodid die Titelverbindung hergestellt.
$^1$H-NMR ($D_2O$): δ 5,35 und 5,14 (2m, 1H); 4,46 (m, 2H); 3,25-3,43 (m, 4H); 2,3-2,8 (m, 4H); 1,2 (t, 3H).

Beispiel 9: N-(3-Aminoxy-2-fluorpropyl)-ethylamin-dihydrochlorid

Ein Gemisch von 1,0 g (3 mMol) N-Ethyl-N-[3-(1-methylethylidenaminooxy)-2-fluorpropyl]-4-toluolsulfonamid in 10 ml Wasser und 20 ml konz. Salzsäure wird 10 h am Rückfluss erhitzt und danach bei Normaldruck auf die Hälfte eingeengt. Nach dem Abkühlen wird das Reaktionsgemisch filtriert, mit Ether gewaschen und zur Trockne eingedampft. Der Rückstand stellt die Titelverbindung dar.
$^1$H-NMR ($D_2O$): δ 1,25 (t, 3H); 3,30 (m, 2H); 3,48 (m, 2H); 4,39 (m, 2H); 5,15 und 5,31 (2m, 1H).

Die Ausgangsverbindungen werden wie folgt hergestellt:

a: N-[3-(1-Methyl-ethylidenaminooxy)-2-fluorpropyl]-4-toluolsulfonamid

Eine Lösung von 0,5 g (2,7 mMol) 3-(1-Methyl-ethylidenaminooxy)-2-fluorpropylaminhydrochlorid in 10 ml Wasser wird unter Rühren nacheinander mit einer Lösung von 0,52 g (2,8 mMol) Toluol-4-sulfochlorid in 10 ml THF und 8,1 ml 1 N NaOH versetzt. Das Reaktionsgemisch wird 6 h bei Raumtemperatur gerührt und mit 100 ml Essigester verdünnt. Die organische Phase wird abgetrennt, mit 50 ml 0,5 N HCl und Wasser gewaschen, über Magnesiumsulfat getrocknet und eingedampft. Der gelbe ölige Rückstand stellt die Ausgangsverbindung a dar.
$^1$H-NMR ($CDCl_3$): δ 1,82 (s, 6H); 2,43 (s, 3H); 3,1-3,4 (m, 2H); 4,0-4,42 (m, 2H); 4,6 und 4,85 (2m, 1H); 4,96 (t, 1H); 7,31 (d, 2H); 7,74 (d, 2H).

b: N-Ethyl-N-[3-(1-methyl-ethylidenaminooxy)-2-fluorpropyl]-4-toluolsulfonamid

Eine Lösung von 0,36 g (1,2 mMol) N-[3-(1-Methyl-ethylidenaminooxy)-2-fluorpropyl]-4-toluolsulfonamid in 2 ml DMF wird mit 0,415 g (3 mMol) Pottasche und 0,13 ml (1,8 mMol) Ethylbromid versetzt und 24 h bei Raumtemperatur gerührt. Danach wird das Reaktionsgemisch mit 20 ml Essigester verdünnt und filtriert. Das Filtrat wird mit Wasser gewaschen, über Magnesiumsulfat getrocknet, filtriert und eingedampft. Der so erhaltene Rückstand stellt die Ausgangsverbindung b dar.
$^1$H-NMR ($CDCl_3$): δ 1,12 (t, 3H); 1,83 (3, 6H); 2,40 (s, 3H); 3,10-3,66 (m, 4H); 4,18 (q, 2H); 4,76 und 5,01 (2m, 1H); 7,27 (d, 2H); 7,66 (d, 2H).

Beispiel 10: N-(3-Aminoxy-2-fluorpropyl)-propylamin-dihydrochlorid

Analog Beispiel 9 wird ausgehend von N-[3-(1-Methyl-ethylidenaminooxy)-2-fluorpropyl]-4-toluolsulfonamid und n-Propyljodid die Titelverbindung hergestellt.
$^1$H-NMR ($D_2O$): δ 5,29 und 5,06 (2m, 1H); 4,43 (m, 2H); 3,21-3,5 (m, 6H); 1,21 (t, 3H).

Beispiel 11: N-(3-Aminoxy-2-fluorpropyl)-n-butylamin-dihydrochlorid

Analog Beispiel 9 wird ausgehend von N-[3-(1-Methyl-ethylidenaminoxy)-2-fluorpropyl]-4-toluolsulfonamid und n-Butyljodid die Titelverbindung hergestellt.
$^1$H-NMR ($D_2O$): δ 5,35 und 5,14 (2m, 1H); 4,46 (m, 2H); 3,25-3,43 (m, 4H); 2,3-2,8 (m, 4H); 1,2 (t, 3H).

Beispiel 12: Kapseln enthaltend 0,25 g Wirkstoff, z.B. eine der Verbindungen der Beispiele 1-2, können wie folgt hergestellt werden:

| Zusammensetzung (für 5000 Kapseln) | |
|---|---|
| Wirkstoff | 1250 g |
| Talk | 180 g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

## Patentansprüche

1. Verbindungen der Formel

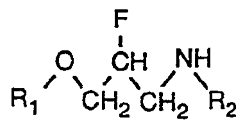

(I),

worin $R_1$ Amino bedeutet oder für einen Rest

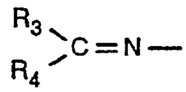

steht, worin $R_3$ Niederalkyl, Hydroxyniederalkyl, Niederalkoxyniederalkyl, Carboxy, Niederalkoxycarbonyl, Phenyl, durch Niederalkyl, Halogenniederalkyl, Hydroxyniederalkyl, Halogen, Hydroxy, Niederalkoxy, Niederalkanoyloxy und/oder Nitro substituiertes Phenyl, Pyridyl, durch Niederalkyl, Hydroxyniederalkyl, Niederalkoxyniederalkyl, Phosphonooxyniederalkyl, Niederalkanoyl, Carboxy, Niederalkoxycarbonyl, Hydroxy, Niederalkoxy, Niederalkanoyloxy, Nitro und/oder Oxido substituiertes Pyridyl oder Chinolyl bedeutet, $R_4$ für Wasserstoff, Niederalkyl, Hydroxyniederalkyl oder Halogenniederalkyl steht oder $R_3$ und $R_4$ zusammen $C_4$-$C_6$-Alkylen oder Benzo-$C_4$-$C_6$-alkylen bedeuten, und $R_2$ geradkettiges $C_1$-$C_4$-Alkyl bedeutet, und Salze davon.

2. Verbindungen der Formel I gemäss Anspruch 1, worin $R_1$ Amino bedeutet oder für einen Rest

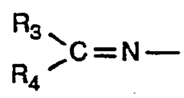

steht, worin $R_3$ Niederalkyl, Hydroxyniederalkyl, Niederalkoxyniederalkyl, Phenyl, durch Hydroxy substituiertes Phenyl, Pyridyl, durch Hydroxy, Niederalkyl, Hydroxyniederalkyl und/oder Phosphonooxyniederalkyl substituiertes Pyridyl, oder Chinolyl bedeutet, $R_4$ Wasserstoff, Niederalkyl oder Halogenniederalklyl bedeutet, oder $R_3$ und $R_4$ zusammen für $C_4$-$C_6$-Alkylen oder Benzo-$C_4$-$C_6$-alkylen stehen, und $R_2$ geradkettiges $C_1$-$C_4$-Alkyl bedeutet, und Salze davon.

3. Verbindungen der Formel I gemäss Anspruch 1, worin $R_1$ Amino bedeutet oder für einen Rest

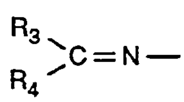

steht, worin $R_3$ Niederalkyl, durch Hydroxy substituiertes Phenyl oder durch Hydroxy, Niederalkyl, Hydroxyniederalkyl und/oder Phosphonooxyniederalkyl substituiertes Pyridyl bedeutet, $R_4$ Wasserstoff oder Niederalkyl bedeutet, und $R_2$ geradkettiges $C_1$-$C_4$-Alkyl bedeutet, und Salze davon.

4. Verbindungen der Formel I gemäss Anspruch 1, worin $R_1$ Amino bedeutet und $R_2$ geradkettiges $C_1$-$C_4$-Alkyl bedeutet, und Salze davon.

5. Pharmazeutisch annehmbare Salze von Verbindungen der Formel I gemäss Anspruch 1.

6. N-(3-Aminoxy-2-fluorpropyl)-methylamin und pharmazeutisch annehmbare Salze davon gemäss Anspruch 1.

7. Pharmazeutisches Präparat enthaltend eine Verbindung der Formel I gemäss Anspruch 1.

8. Eine Verbindung der Formel I gemäss Anspruch 1 zur Anwendung in einem Verfahren zur therapeutischen Behandlung des menschlichen oder tierischen Körpers.

9. Verwendung einer Verbindung der Formel I gemäss Anspruch 1 zur Herstellung von pharmazeutischen Präparaten.

10. Verfahren zur Herstellung von Verbindungen der Formel I gemäss Anspruch 1, dadurch gekennzeichnet, dass man
(a) eine Verbindung der Formel II

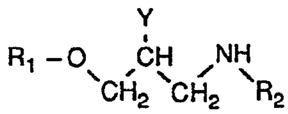

(II)

worin $R_1$ und $R_2$ die unter Formel I angegebene Bedeutung haben und Y eine in Fluor überführbare oder durch Fluor austauschbare Gruppe ist, mit einem Fluorierungsmittel umsetzt, oder
(b) zur Herstellung von Verbindungen der Formel I, worin $R_1$ Amino bedeutet, in einer Verbindung der Formel

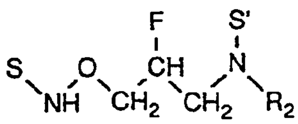

(III)

worin $R_2$ die unter Formel I angegebene Bedeutung hat und S und S' unabhängig voneinander für eine Aminoschutzgruppe oder Wasserstoff stehen, wobei mindestens eine der Gruppen S und S' eine Aminoschutzgruppe bedeutet, die Aminoschutzgruppe(n) abspaltet, oder
(c) zur Herstellung von Verbindungen der Formel I, worin $R_1$ Amino bedeutet, eine Verbindung der Formel

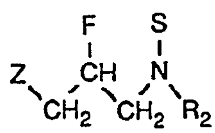

(IV)

worin $R_2$ die unter Formel I angegebene Bedeutung hat, Z Hydroxy oder eine nucleofuge Abgangsgruppe bedeutet und S eine Aminoschutzgruppe oder Wasserstoff bedeutet, mit gegebenenfalls N-geschütztem Hydroxylamin umsetzt und gegebenenfalls die Aminoschutzgruppe abspaltet, oder
(d) eine Verbindung der Formel

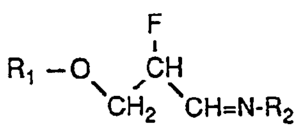

(V)

worin $R_1$ und $R_2$ die unter Formel I angegebene Bedeutung haben, reduziert; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung in ein Salz umwandelt, und/oder ein erhaltenes Gemisch von isomeren Verbindungen der Formel I in die einzelnen Isomeren auftrennt.

11. Die nach dem Verfahren gemäss Anspruch 10 erhältlichen Verbindungen.

**Patentansprüche für folgenden Vertragsstaaten: ES und GR**

**1.** Verfahren zur Herstellung einer Verbindung der Formel

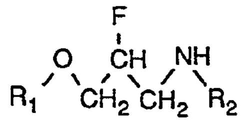

(I),

worin $R_1$ Amino bedeutet oder für einen Rest

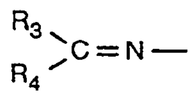

steht, worin $R_3$ Niederalkyl, Hydroxyniederalkyl, Niederalkoxyniederalkyl, Carboxy, Niederalkoxycarbonyl, Phenyl, durch Niederalkyl, Halogenniederalkyl, Hydroxyniederalkyl, Halogen, Hydroxy, Niederalkoxy, Niederalkanoyloxy und/oder Nitro substituiertes Phenyl, Pyridyl, durch Niederalkyl, Hydroxyniederalkyl, Niederalkoxyniederalkyl, Phosphonooxyniederalkyl, Niederalkanoyl, Carboxy, Niederalkoxycarbonyl, Hydroxy, Niederalkoxy, Niederalkanoyloxy, Nitro und/oder Oxido substituiertes Pyridyl oder Chinolyl bedeutet, $R_4$ für Wasserstoff, Niederalkyl, Hydroxyniederalkyl oder Halogenniederalkyl steht oder $R_3$ und $R_4$ zusammen $C_4$-$C_6$-Alkylen oder Benzo-$C_4$-$C_6$-alkylen bedeuten, und $R_2$ geradkettiges $C_1$-$C_4$-Alkyl bedeutet, und ihrer Salze, dadurch gekennzeichnet, dass man

(a) eine Verbindung der Formel II

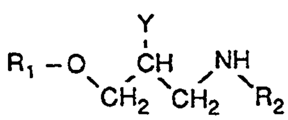

(II)

worin $R_1$ und $R_2$ die unter Formel I angegebene Bedeutung haben und Y eine in Fluor überführbare oder durch Fluor austauschbare Gruppe ist, mit einem Fluorierungsmittel umsetzt, oder

(b) zur Herstellung von Verbindungen der Formel I, worin $R_1$ Amino bedeutet, in einer Verbindung der Formel

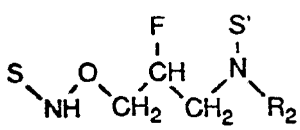

(III)

worin $R_2$ die unter Formel I angegebene Bedeutung hat und S und S' unabhängig voneinander für eine Aminoschutzgruppe oder Wasserstoff stehen, wobei mindestens eine der Gruppen S und S' eine Aminoschutzgruppe bedeutet, die Aminoschutzgruppe(n) abspaltet, oder

(c) zur Herstellung von Verbindungen der Formel I, worin $R_1$ Amino bedeutet, eine Verbindung der Formel

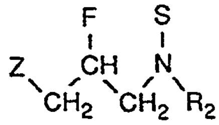

(IV)

worin $R_2$ die unter Formel I angegebene Bedeutung hat, Z Hydroxy oder eine nucleofuge Abgangsgruppe bedeutet und S eine Aminoschutzgruppe oder Wasserstoff bedeutet, mit gegebenenfalls N-geschütztem Hydroxylamin umsetzt und gegebenenfalls die Aminoschutzgruppe abspaltet, oder (d) eine Verbindung der Formel

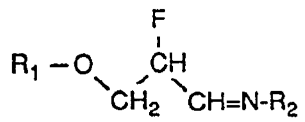

(V)

worin $R_1$ und $R_2$ die unter Formel I angegebene Bedeutung haben, reduziert; und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung in ein Salz umwandelt, und/oder ein erhaltenes Gemisch von isomeren Verbindungen der Formel I in die einzelnen Isomeren auftrennt.

2. Verfahren gemäss Anspruch 1 zur Herstellung einer Verbindung der Formel I worin $R_1$ Amino bedeutet oder für einen Rest

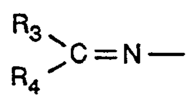

steht, worin $R_3$ Niederalkyl, Hydroxyniederalkyl, Niederalkoxyniederalkyl, Phenyl, durch Hydroxy substituiertes Phenyl, Pyridyl, durch Hydroxy, Niederalkyl, Hydroxyniederalkyl und/oder Phosphonooxyniederalkyl substituiertes Pyridyl, oder Chinolinyl bedeutet, $R_4$ Wasserstoff, Niederalkyl oder Halogenniederalklyl bedeutet, oder $R_3$ und $R_4$ zusammen für $C_4$-$C_6$-Alkylen oder Benzo-$C_4$-$C_6$-alkylen stehen, und $R_2$ geradkettiges $C_1$-$C_4$-Alkyl bedeutet, und ihrer Salze.

3. Verfahren gemäss Anspruch 1 zur Herstellung einer Verbindung der Formel I, worin $R_1$ Amino bedeutet oder für einen Rest

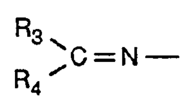

steht, worin $R_3$ Niederalkyl, durch Hydroxy substituiertes Phenyl oder durch Hydroxy, Niederalkyl, Hydroxyniederalkyl und/oder Phosphonooxyniederalkyl substituiertes Pyridyl bedeutet, $R_4$ Wasserstoff oder Niederalkyl bedeutet, und $R_2$ geradkettiges $C_1$-$C_4$-Alkyl bedeutet, und ihrer Salze.

4. Verfahren gemäss Anspruch 1 zur Herstellung einer Verbindung der Formel I, worin $R_1$ Amino bedeutet und $R_2$ geradkettiges $C_1$-$C_4$-Alkyl bedeutet, und ihrer Salze.

5. Verfahren gemäss Anspruch 1 zur Herstellung von N-(3-Aminoxy-2-fluorpropyl)-methylamin und pharmazeutisch annehmbarer Salze davon.

6. Verfahren zur Herstellung einer pharmazeutischen Zusammensetzung enthaltend als Wirkstoff mindestens eine der in den Ansprüche 1-5 definierten Verbindungen, dadurch gekennzeichnet, dass man diesen Wirkstoff mit mindestens einem pharmazeutisch anwendbaren Trägermaterial verarbeitet.

7. Verfahren nach Anspruch 1, dadurch gekennzeichnet, dass man N-(3-Aminoxy-2-fluorpropyl)-methylamin und pharmazeutisch annehmbare Salze davon herstellt.